(19) Europäisches Patentamt
European Patent Office
Office européen des brevets

(11) Veröffentlichungsnummer : **0 417 588 B1**

(12) # EUROPÄISCHE PATENTSCHRIFT

(45) Veröffentlichungstag der Patentschrift :
**19.08.92 Patentblatt 92/34**

(51) Int. Cl.$^5$ : **A61K 47/48**

(21) Anmeldenummer : **90116864.1**

(22) Anmeldetag : **03.09.90**

(54) **Verfahren zur Herstellung eines komplexierten Arzneimittels.**

(30) Priorität : **14.09.89 DE 3930733**

(43) Veröffentlichungstag der Anmeldung :
**20.03.91 Patentblatt 91/12**

(45) Bekanntmachung des Hinweises auf die Patenterteilung :
**19.08.92 Patentblatt 92/34**

(84) Benannte Vertragsstaaten :
**AT BE CH DE DK ES FR GB IT LI NL SE**

(56) Entgegenhaltungen :
**CH-A- 664 284**
**DE-A- 1 944 693**
**DE-A- 2 752 705**
**US-A- 4 539 199**

(56) Entgegenhaltungen :
**CHEMICAL ABSTRACTS, Band 84, 1976, Seite 334, Zusammenfassung Nr. 126737y, Columbus, Ohio, US; N.A. ELGINDY: "Molecularentrapment of cationic drugs by Carbopol 934", & CAN. J. PHARM. SCI. 1976, 11(1), 32-4**

(73) Patentinhaber : **RÖHM GMBH**
**Kirschenallee**
**W-6100 Darmstadt (DE)**

(72) Erfinder : **Vincze, Andreas, Dr.**
**Mauerkircher Strasse 33**
**W-8000 München 80 (DE)**
Erfinder : **Assmus, Manfred**
**Erbsengasse 9**
**W-6101 Bickenbach (DE)**
Erfinder : **Lehmann, Klaus, Dr.**
**Schillerstrasse 85**
**W-6101 Rossdorf 1 (DE)**
Erfinder : **Petereit, Hans-Ulrich**
**Lortzingstrasse 22**
**W-6100 Darmstadt (DE)**



Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung eines komplexierten Arzneimittels aus einem ionogenen Wirkstoff durch Umsetzung des Wirkstoffes mit einem komplementär ionogenen partikelförmigen Polymeren. In dem komplexierten Arzneimittel liegt wenigstens ein Teil des ionogenen Wirkstoffes in Form eines salzartigen Komplexes mit dem komplementär ionogenen Polaren vor. Solche Komplexe haben gegenüber dem ionogenen Wirkstoff selbst oft Vorteile, wie verzögerte Wirkstoffabgabe, verminderten Eigengeschmack oder gegebenenfalls höhere Stabilität.

Mit dem Ausdruck "ionogen" wird hier zum Ausdruck gebracht, daß die ionogene Verbindung, also der Wirkstoff oder das Polder in einen ionischen Zustand überzugehen vermag. Wenn die Verbindung in Form eines wasserlöslichen Salzes vorliegt, genügt in der Regel die Zugabe einer ausreichenden Menge an Wasser, um eine wenigstens teilweise Auflösung unter Bildung von hydratisierten Ionen zu bewirken. Liegt die ionogene Verbindung ins Form einer Säure oder Base vor, so bildet sie durch Umsetzung mit einer komplementär ionogenen Verbindung Ionen; beispielsweise bildet ein basischer Wirkstoff durch Umsetzung mit einer Säure ein Salz, welches aus einem Kation des Wirkstoffes und dem Anion der Säure besteht. Für die Zwecke der Erfindung ist es von untergeordneter Bedeutung auf welchem Wege die ionogenen Verbindungen in den ionischen Zustand übergehen.

Unter einem komplexierten Arzneimittel wird im Sinne der Erfindung eine Verbindung verstanden, die Ionen des Wirkstoffes und die entsprechenden Gegenionen des Polymeren enthält, also beispielsweise Kationen eines stickstoffhaltigen basischen Wirkstoffes und Anionen eines Carboxylatgruppen enthaltenden Polymeren. Von komplexierten Arzneimitteln wird vor allem dann gesprochen, wenn sie in Wasser schwer- oder unlöslich sind, jedoch ist weder dieses noch ein nachweisbarer chemischer Bindungszustand, der über die ionische Bindung hinausgeht, eine zwingende Voraussetzung für die Anwendung der Erfindung.

Stand der Technik

Komplexierte Arzneimittel werden gemäß DE-A 27 52 705 hergestellt, indem man eine wäßrige Lösung eines ionogenen Polymeren mit einer wäßrigen Lösung eines komplementär ionogenen Wirkstoffes umsetzt, wobei das Umsetzungsprodukt als schwerlöslicher Komplex ausfällt. Das Herstellungverfahren erfordert eine Vielzahl von Arbeitsschritten, nämlich die Herstellung der wäßrigen Lösungen des Wirkstoffes und des Polymeren, deren allmähliche Vermischung und Umsetzung, schließlich die Filtration, Auswaschung, Trocknung und Vermahlung des Präzipitats, bis ein zur Weiterverarbeitung geeignetes Präparat vorliegt.

In gleichartiger Weise wird gemäß CH-A 664 284 ein Chloroquin-Präparat erzeugt, indem eine wäßrige Lösung von Chloroquin-diphosphat mit einer Lösung eines Copolymeren von Natriummethacrylat und Ethylacrylat unter Bildung eines unlöslichen Komplexes umgesetzt wird.

Nach der Lehre der US-A 3 629 392 werden ein wäßriger Latex, der ein partikelförmiges ionogenes Polymer enthält, mit einer wäßrigen Dispersion eines komplementär ionogenen, wenigstens begrenzt wasserlöslichen Wirkstoffes in einem flüssigen Reaktionsgemisch miteinander umgesetzt, wobei im Falle eines schwerlöslichen Wirkstoffes der jeweils gelöste Anteil mit dem Polymerlatex reagiert, bis die Gesamtmenge des Wirkstoffes umgesetzt ist. Wenn das komplexierte Arneimittel nicht von selbst ausfällt, muß der Latex nach der Umsetzung durch Koagulationsmittel ausgefällt und das komplexierte Arzneimittel abfiltriert werden. Dabei müssen Maßnahmen ergriffen werden, um zu verhindern, daß nicht umgesetzte Anteile des Wirkstoffes oder nicht koagulierte Anteile des Latex mit dem Filtrat verlorengehen. Es ist auch möglich, den umgesetzten Latex völlig einzutrocknen, wobei unter Einsatz hoher Energiemengen große Wassermengen verdampft werden müssen. Dazu müssen in einem eigenen Verfahrensschritt besondere Verdampfungsapparaturen, wie Sprühtrockner, Gefriertrockner oder Vakuumverdampfer, eingesetzt werden.

Auch in der US-A 4 539 199 wird die Umsetzung eines salzartigen ionogenen Wirkstoffes, wie Epinephrinhydrochlorid, mit einem Latex eines anionischen Polaren unter Zusatz einer Alkalibase beschrieben, wobei das entstehende komplexierte Arzneimittel ausfällt und abfiltriert wird. Hier - wie im vorigen Verfahren - wird die Herstellung eines retardierten Präparats angestrebt.

Allen erwähnten Verfahren ist gemeinsam, daß die Umsetzung in einem flüssigen Reaktionsgemisch vorgenommen wird, das die ionogenen Ausgangsstoffe gegebenenfalls in dispergierter Form enthalten kann. Das Umsetzungsprodukt ist demnach in jedem Fall ein flüssiges System, das - sofern es nicht zur Herstellung eines Flüssigpräparats verwendet wird - in einem oder mehreren nachfolgenden Verfahrensschritten in ein Festprodukt übergeführt werden muß.

Aufgabe und Lösung

Es ist das Ziel der Erfindung, die Umwandlung eines ionogenen Wirkstoffes in ein komplexiertes Trokkenprodukt zu vereinfachen bzw. mit vermindertem Energieeinsatz durchzuführen. Zu diesem Zweck wird erfindungsgemäß eine angefeuchtete pulverförmige Mischung aus dem Wirkstoff und dem partikelförmigen Polymeren hergestellt und getrocknet.

Die angefeuchtete pulverförmige Mischung enthält so viel Wasser in Form von an der Partikeloberfläche haftender Feuchtigkeit, daß wenigstens eine oberflächliche Umsetzung zwischen dem Wirkstoff und dem partikel förmigen Polymeren stattfindet. Es ist möglich, aber nicht unumgänglich notwendig, daß im Laufe der Umsetzung der gesamte Wirkstoff vorübergehend in Lösung geht und jeweils der gelöste Anteil mit dem partikelförmigen Polymeren reagiert und sich dann in Salzform wieder abscheidet. Eine genaue Untersuchung über den Ablauf und das Ausmaß der Umsetzung ist von den Erfindern nicht durchgeführt worden. Es wurde jedoch festgestellt, daß die mit der Salzbildung verbundenen Wirkungen eintreten, nämlich eine mehr oder weniger ausgeprägte Retardwirkung und vor allem eine spürbare Überdeckung bzw. Unterdrückung des eventuellen Bittergeschmackes des Wirkstoffes. In manchen Fällen läßt sich auch eine Stabilisierung des Wirkstoffes gegen Oxydation und andere zersetzende Einflüsse, die unter den gleichen Umständen den freien Wirkstoff schädigen würden, feststellen.

Überraschenderweise werden diese Wirkungen erzielt, ohne daß ein flüssiges Reaktionsgemisch gebildet wird, selbst wenn der Wirkstoff pulverförmig eingesetzt wird. Die Mischung bleibt während der Dauer der Umsetzung zwischen dem Wirkstoff und dem partikel förmigen Polymeren stets krümelig bis breiartig, jedoch nicht fließfähig.

Vorteile und Anwendung der Erfindung

Die Mischung enthält wesentlich weniger Wasser als die wäßrigen Lösungen oder Aufschlemmungen, in denen bisher Umsetzungen von ionogenen Wirkstoffen mit komplementär ionogenen Polymeren durchgeführt wurden. Das trifft vor allem dann zu, wenn der Wirkstoff in Pulverform eingesetzt wird, so daß diese Ausführungsform bevorzugt ist. Daher ist auch weniger Wasser zu entfernen, wenn nach erfolgter Umsetzung ein Trockenprodukt hergestellt wird. Besondere Verfahrensmaßnahmen zur Trennung der flüssigen und festen Bestandteile der Mischung sind entbehrlich. Vielmehr kann die Mischung als solche getrocknet werden. In der Regel kann dies auf Horden in einem Trockenschrank oder in Wirbelschichtgeräten geschehen. Mit entsprechender Ausrüstung kann man die feuchte Mischung gegebenenfalls durch direktes Erwärmen in dem gleichen Reaktionsapparat trocknen, in dem sie hergestellt und umgesetzt worden ist. Die Verminderung des umgebenden Luftdruckes beschleunigt die Trocknung.

Das 50 erhaltene Trockenprodukt kann in manchen Fällen als solches als Arzneimittel verwendet werden. Vorzugsweise wird es in bekannter Weise zu üblichen galenischen Darreichungsformen verarbeitet. Erforderlichen falls können die beim Trocknen anfallenden Körner in einer geeigneten Mühle zu einem Pulver mit Korngrößen im Bereich von 1 bis 1000 Mikrometer zerkleinert werden. Das 50 erhaltene Pulver wird beispielsweise zu Tabletten verpreßt oder zu Pillen geformt oder in einem Sirup suspendiert oder in kau- oder lutschbare Arzneiformen (Kautabletten, Kaugummi) eingearbeitet.

In dem erfindungsgemäß hergestellten Trockenprodukt liegt der enthaltene Wirkstoff weitgehend, in der Regel zu mehr als 90 %, in Form eines Komplexsalzes mit dem Polymer vor. Der Anteil des nicht komplex gebundenen Wirkstoffes läßt sich leicht bestimmen, wenn man das Produkt in Wasser aufnimmt, filtriert und den Wirkstoffgehalt im Filtrat ermittelt. Der nicht komplexierte Anteil kann erforderlichenfalls auf die gleiche Weise aus dem Produkt entfernt werden, ebenso lösliche Nebenprodukte der Komplexbildung, wie beispielsweise Natriumbromid, das bei der Umsetzung von Arzneistoffbromiden mit anionischen Polymeren in Gegenwart von Natriumcarbonat entsteht.

Der Polymer-Wirkstoff-Komplex selbst ist schwerlöslich oder praktisch unlöslich, steht aber bei Anwesenheit einer wäßrigen Phase nach dem Ionenaustauschprinzip mit dem gelöstem Wirkstoff im Gleichgewicht. Der Wirkstoff wird daher in vitro wie in vivo in dem Maße wieder freigesetzt, wie er der wäßrigen Phase entzogen oder wie diese ersetzt wird. Er ist auf diese Weise vollständig bioverfügbar. Die Freisetzungsgeschwindigkeit hängt allerdings von einer Mehrzahl von Parametern ab, z.B. dem pH-Wert und der Ionenstärke der umgebenden Flüssigkeit. Hohe Ionenstärke und niedrige pH-Werte beschleunigen die Freigabe basischer Wirkstoffe. Abb. 1 stellt die Abhängigkeit der Freisetzung des Wirkstoffes Verapamil von der Ionenstärke des wäßrigen Mediums dar. Außerdem beeinflußt die Teilchengröße des Arzneistoff-Polymer-Komplexes die Wirkstoffabgaberate; grobe Partikel über 0,6 mm setzen den Wirkstoff langsamer frei als feinere Pulver unter 0,5 mm Korngröße, wie in Abb. 2 veranschaulicht ist.

Die Überdeckung des unangenehmen, insbesondere bitteren Geschmackes eines Wirkstoffes ist eines der wesentlichen Ziele des Verfahrens der Erfindung. Als Maß für die Geschmacksintensität kann der Bitterwert nach DAB 9 herangezogen werden. Die Verminderung der bitteren Geschmackempfindung läuft mit dem gebundenen Anteil ungefähr proportional; während z.B. reines Verapamilhydrochlorid einen Bitterwert über 100.000 hat, liegt der Wert für den Komplex mit 90 % gebundenem Wirkstoff unter 5000 und mit 97 % gebundenem Wirkstoff unter 1000. Ein Bitterwert von 1000 ist für die pharmazeutische Praxis in der Regel ausreichend. Der dafür erforderliche Grad der Komplexierung hängt vom Bitterwert des reinen Wirkstoffes und von seiner Konzentration in der Darreichungsform ab.

Die erfindungsgemäß hergestellten Wirkstoffkomplexe können direkt als Pulver, gegebenenfalls nach Mahlen, appliziert werden. Sie lassen sich jedoch auch mit üblichen pharmazeutischen Hilfsstoffen kombinieren und zu anspruchsvolleren Arzneiformen verarbeiten. In Kombination mit Bindemitteln, wie mikrokristalliner Cellulose oder Zuckern, mit Sprengmitteln, wie Stärke oder ihren Derivaten, vernetztem Polyvinylpyrrolidon oder quellenden Cellulosederivaten, und mit Gleitmitteln, wie Talkum oder Magnesiumstearat, lassen sich Tabletten pressen, die nach der Applikation rasch zerfallen. In wäßriger Suspension lassen sich die Arzneistoff-Polymer-Komplexe mit üblichen Gelbildnern stabilisieren, sofern deren ionischer Charakter nicht zu einer Erhöhung des ungebundenen Wirkstoffanteils führt; als Beispiele seien Xantan und Hydroxypropylmethylcellulose genannt. Die optimierten Suspensionen sind geschmacksneutral und lagerstabil. Sie können durch geeignete Zusätze aromatisiert werden.

Herstellung des komplexierten Arzneimittels

Das Verfahren der Erfindung ist am besten mit pulverförmigen festen Wirkstoffen durchführbar, jedoch können auch flüssige Wirkstoffe eingesetzt werden. Vorzugsweise wird die Erfindung in der Weise ausgeführt, daß der Wirkstoff in Form eines Salzes mit einem organischen oder anorganischen Gegenion eingesetzt wird und daß der Mischung zusätzlich eine niedermolekulare Säure oder Base zur Neutralisation des Gegenions des Wirkstoffes zugesetzt wird.

Der ionogene Wirkstoff kann in Form einer freien Säure, insbesondere einer organischen Carbonsäure vorliegen; als Beispiele seien Acetylsalicylsäure, nichtsteroidale Antirheumatika, wie Ibuprofen oder Ketoprofen, und Barbiturate genannt. In diesem Falle läßt sich der Wirkstoff unmittelbar mit einem basischen partikelförmigen Polymer umsetzen. Liegt er aber in Form eines Salzes einer Säure, beispielsweise als Natrium-, Kalium-, Calcium-oder Magnesiumsalz vor, so wird es bevorzugt, die Umsetzung in Gegenwart einer Säure vorzunehmen, die stärker als die dem Wirkstoff zugrundeliegende Säure ist. Die Säure kann in dem zum Anfeuchten der pulverförmigen Mischung verwendeten Wasser enthalten sein oder vorteilhafter in fester Form zugesetzt werden; dafür eignen sich beispielsweise saure Salze, wie Natrium- oder Ammoniumhydrogensulfat, Natriumdihydrogen-phosphat, oder feste organische Säuren ausreichender Stärke, wie Citronensäure oder Weinsäure.

Bevorzugt wird das Verfahren der Erfindung mit einer basischen organischen Stickstoffverbindung oder deren Salz mit einer niedermolekularen Säure ausgeführt. Viele der zu dieser Substanzklasse gehörenden Wirkstoffe haben einen unangenehmen Bittergeschmack, so daß das Verfahren hauptsächlich zur Geschmackskaschierung angewendet wird. Das trifft für viele allgemein bekannte Wirkstoffe aus den Klassen der Antitussiva, Herz-Kreislauf-Mittel, Antibiotika und Chemotherapeutika zu. Sofern der Wirkstoff in der Basenform vorliegt, kann er unmittelbar mit einem sauren Polymer umgesetzt werden. Liegt er dagegen in Form eines Salzes des basischen Wirkstoffes mit einer niedermolekularen Säure, wie Salzsäure, Bromwasserstoffsäure, Schwefelsäure, Phosphorsäure, Essigsäure, Milchsäure oder Maleinsäure vor, so ist es zweckmäßig, die Umsetzung in Gegenwart einer Base durchzuführen. Als Basen eignen sich feste basische Verbindungen in Pulverform, wie die Hydroxyde der Alkali-oder Erdalkalimetalle oder deren basische Salze oder Carbonate. Eine wenigstens begrenzte Wasserlöslichkeit dieser Basen ist von Vorteil. Besonders geeignet ist pulverförmiges Natriumcarbonat.

Das zu dem Wirkstoff komplementär ionogene Polymer kann sauren oder basischen Charakter haben, der durch einen Gehalt des Polymers an Carboxyl- oder Sulfonyl-Gruppen bzw. an basischen Aminogruppen hervorgerufen wird. Da das Polymer in Partikelform eingesetzt wird, ist es wichtig, daß die sauren oder basischen Gruppen an der Oberfläche der Polymerpartikel in ausreichender Menge zugänglich sind. Dagegen ist ein Gehalt an solchen Gruppen im Innern der Polymerpartikel nicht erforderlich, wenn auch herstellungsbedingt in der Regel vorhanden. Der Anteil der an der Oberfläche zugänglichen Gruppen ist umso höher, je feinteiliger das Polymer ist. Gut geeignet sind sprühgetrocknete Emulsionspolymerisate. Die Partikelgröße kann zwischen etwa 50 nm und 1 mm liegen. Sind die Partikel größer, so steigt der Bedarf an Polymersubstanz, mit dem die erwünschte Wirkung erzielbar ist, unverhältnismäßig stark an.

Der für die Komplexbildung erforderliche Anteil an funktionellen Gruppen hat zur Folge, daß das Polymerisat in der Regel in bestimmten pH-Bereichen wasserlöslich ist. Beispielsweise geht ein Copolymer aus 50 Gew.-% Methacrylsäure und 50 Gew.-% Ethylacrylat oder Methylmethacrylat bei pH 5,5 bis 6,0 in Lösung. Liegt der Methacrylsäureanteil bei 30 Gew.-%, so tritt erst oberhalb pH 7 Auflösung ein. Bei der Applikation können die Polymeren partiell in Lösung gehen, während der Polymer-Wirkstoff-Komplex bis zur Abgabe des Wirkstoffes üblicherweise ungelöst bleibt.

Im allgemeinen hängt die Eignung des Polymeren nicht von seiner Herstellungsweise ab. Allerdings haben die Korngröße und die Struktur des eingesetzten Polymeren einen deutlichen Einfluß auf die Freisetzungsgeschwindigkeit. Feinteilige Polymere sind vorteilhaft, wenn auf eine rasche Freisetzung des Wirkstoffes Wert gelegt wird. In diesem Falle sind Polymerpartikel in Form eines wäßrigen Latex besonders vorteilhaft. Typische Latizes enthalten Teilchen einer (mittleren) Größe von 50 bis 2000 nm. Die Wasserphase des Latex kann zum Anfeuchten der pulverförmigen Mischung dienen. Damit keine flüssige Reaktionsmischung entsteht, muß der Feststoffgehalt des Latex ausreichend hoch sein, beispielsweise 40 bis 65 Gew.-%, es sei denn, daß daneben trockenes Polymerpulver oder andere trockene Zusatzstoffe mitverwendet werden. Man kann auch sprühgetrocknete Emulsionspolymerisate einsetzen, die in der Regel aus lockeren kugeligen Aggregaten im Korngrößenbereich von 0,01 bis 2 mm vorliegen. In diesem Falle wird die Mischung mit Wasser angefeuchtet. Feinteilige Perlpolymerisate sind in gleicher Weise verwendbar.

Eine bevorzugte Gruppe von Polymeren leitet sich von äthylenisch ungesättigten, radikalisch polymerisierbaren Monomeren ab, von denen wenigstens ein Teil ionogen sein muß. Als Monomere mit sauren Gruppen eignen sich äthylenisch ungesättigte Carbonsäuren, wie Malein-, Fumar-, oder Itakonsäure oder deren Halbester und bevorzugt Acryl- und/oder Methacrylsäure. Bevorzugt verwendet man ein Mischpolymerisat aus 10 bis 90 Gew.-% einer äthylenisch ungesättigten, radikalisch polymerisierbaren Carbonsäure mit wenigstens einem nichtionogenen, wasserunlöslichen äthylenisch ungesättigten, radikalisch polymerisierbaren Comonomer. Ist der ionogene Wirkstoff eine Säure, so wird ein partikelförmiges Polymer eingesetzt, das als komplementär ionogene Gruppen seitenständige Aminogruppen enthält. Dieses leitet sich vorzugsweise von Monomeren mit basischen Gruppen ab. Als solche kommen bevorzugt Alkylester und Alkylamide der Acryl- und/oder Methacrylsäure in Betracht, die am Alkylrest eine primäre, sekundäre oder tertiäre Aminogruppe tragen. Der Alkylrest kann beispielsweise 2 bis 8 Kohlenstoffatome enthalten und ist vorzugsweise verzweigt, beispielsweise ein 2,2-Dimethylpropylrest. Tertiäre Aminogruppe stören die radikalische Polymerisation am wenigsten und sind deshalb bevorzugt. Beispiele geeigneter basischer Monomerer sind:

2-Dimethylamino-ethyl-acrylat und -methacrylat
3- (N, N-Dimethylamino)-propyl-acrylat und -methacrylat,
4-(N, N-Dimethylamino)-butyl-acrylat und -methacrylat,
3-(N,N-Dimethylamino)-propyl-acrylamid und -methacrylamid,
Triethanolamin-monoacrylat und -monomethacrylat,
2-(Dimethylaminoethyloxy)-ethyl-acrylat und -methacrylat,
2-Imidazolyl-ethyl-acrylat und -methacrylat,
2-Piperazinyl-ethyl-acrylat und -methacrylat,
2-Piperazinyl-ethyl-acrylamid und -methacrylamid,
N,N-Dimethylamino-neopentyl-acrylat und -methacrylat,
N,N-Dimethylamino-neopentyl-acrylamid und -methacrylamid,
(1,2,2,6,6-Pentamethyl-piperidyl-4)-acrylat und -methacrylat
3-Morpholino-propyl-acrylamid und -methacrylamid, 2-Morpholino-ethyl-acrylat und -methacrylat,
2-(N,N-Dibutylamino-ethyl-acrylat und -methacrylat,
4-Diethylamino-1-methyl-butyl-acrylamid und -methacrylamid.

Das Polymer kann gegebenenfalls allein aus den genannten ionogenen Monomeren aufgebaut sein. In vielen Fällen ist es vorteilhafter, wenn am Aufbau des Polymers auch nichtionogene Monomere beteiligt sind, deren Anteil beispielsweise zwischen 10 und 90 Gew.-%, insbesondere 30 bis 70 Gew.-%, betragen kann. Als Beispiele sind Alkylester und Hydroxyalkylester der Acryl- und/oder Methacrylsäure mit 1 bis 4 Kohlenstoffatomen im Alkylrest, entsprechende Ester der Malein-, Fumar- und Itakonsäure, Acryl- und/oder Methacrylamide, Acryl- und/oder Methacrylnitril, Vinylester niederer aliphatischer Carbonsäuren, Ethylen, Propylen, Styrol, Vinylchlorid oder Vinylpyrrolidon, zu nennen.

Diese Comonomeren haben in vielen Fällen einen deutlichen Einfluß auf die physikalisch-chemischen Eigenschaften der Polymerisate. Zwar haben viele dieser Eigenschaften keinen unmittelbaren Einfluß auf die Komplexbildungsfähigkeit, jedoch können sie die Beschaffenheit der angefeuchteten Masse oder des getrockneten Produktes beeinflussen und insofern für die technische Durchführbarkeit des Verfahrens von Bedeutung sein. Das trifft z.B. für die Härte und für die Glasübergangstemperatur zu. Die Abhängigkeit dieser und anderer Eigenschaften von der Zusammensetzung eines Polymerisats ist allgemein bekannt und vom Fachmann zweckentsprechend anzuwenden. Für den Einsatz in modernen Granuliergeräten eignen sich besonders härtere Polymerisate, wie z.B. Mischpolymerisate der Methacrylsäure mit 50 Gew.-% Methylmethacrylat, das eine Glasübergangstemperatur von 180°C hat, während entsprechende Mischpolymerisate mit 50 Gew.-% Ethylacrylat eine Glasübergangstemperatur von 120°C haben und zähere Massen ergeben, die schwerer zu durchmischen sind. Hochmolekulare Polyacrylsäure vom Carbopol-Typ eignet sich zwar zur Komplexierung von basischen Wirkstoffen, bereitet jedoch in manchen Fällen infolge ihrer hohen Wasserlöslichkeit und ihres hohen Molekulargewichtes Probleme durch hohe Klebrigkeit. Diese kann gegebenenfalls durch inerte Füllstoffe, wie Lactose oder Cellulosepulver, vermindert werden.

Mit besonderem Vorteil werden Polymere eingesetzt, die sich auf dem Gebiet der Galenik als Überzugsmittel oder Matrixmaterial für Arzneiformen bereits bewährt haben und für diese Zwecke behördlich zugelassen sind. Das trifft in erster Linie für die Polymerisate der Acryl- und/oder Methacrylsäure und für deren Copolymerisate mit 10 bis 80 Gew.-% an niederen Alkylestern dieser Säuren (Alkylreste mit 2 bis 4 Kohlenstoffatomen) zu, die unter der Handelsbezeichnung EUDRAGIT (Röhm GmbH) allgemein bekannt sind.

Um eine vollständige Umsetzung des ionogenen Wirkstoffes mit dem komplementär ionogenen Polymer zu bewirken, müßten beide Reaktionspartner in äquimolaren Mengen eingesetzt werden. Das gilt auch für die zusätzlich mitverwendete Säure oder Base, wenn der Wirkstoff in Form eines Salzes mit einem organischen oder anorganischen Gegenion eingesetzt wird. Wenn in der pulverförmigen Reaktionsmischung ein vollständiger Umsatz des eingesetzten Wirkstoffes nicht erreicht, aber zur Erzielung der angestrebten Wirkungen angestrebt wird, kann man von den äquimolaren Mengenverhältnissen abweichen; beispielsweise zu einem Mol- bzw. Äquivalenzverhältnis des ionogenen Wirkstoffes zu dem komplementär ionogenen Polymer von etwa zwischen 1 : 2 und 1 : 10. Die Säure oder Base kann zu dem ionogenen Wirkstoff in einem Mol-bzw. Äquivalenzverhältnis zwischen 1 : 1 und 2 : 1 stehen.

Die Umsetzung ist umso vollständiger, je mehr Wasser zur Anfeuchtung des Reaktionsgemisches eingesetzt wird. Daher kann das Mischungsverhältnis umso näher an den äquimolaren Mengen liegen, je höher der Wassergehalt ist. Der Anteil des Wassers liegt vorteilhaft etwa bei 20 bis 80 Gew.-% des feuchten Reaktionsgemisches, insbesondere im Bereich von 30 bis 50 Gew.-%. Liegt der Wassergehalt niedriger, so ist der Grad der Umsetzung zu gering und die stabilisierende, retardierende oder geschmackskaschierende Wirkung tritt nicht oder erst nach einer langen Umsetzungsdauer im erforderlichen Ausmaß ein. Bei einem höheren Wassergehalt besteht die Gefahr, daß die Mischung klebrig wird und ihre krümelig-pulverförmige Beschaffenheit verliert. Bei noch höherem Wassergehalt würde die Mischung allmählich den Charakter eines flüssigen Gemisches annehmen und dann einen unverhältnismäßig hohen Energieaufwand und Zeitbedarf für die Trocknung benötigten.

Die Beschaffenheit der angefeuchteten Pulvermischung hängt nicht nur von ihrem Wassergehalt ab, sondern auch von anderen physikalisch-chemischen Eigenschaften der Bestandteile. Kleine Partikel im Bereich von 10 bis 50 Mikrometer binden oberflächlich mehr Wasser als solche im Bereich von 0,5 bis 1 mm. Bei gleichem Wassergehalt können daher gröbere Partikel eine breiige Masse bilden, während feinere Pulver eine körnige Masse ergeben. Mikronisierte Partikel unterhalb 5 Mikrometer sind oft schwer benetzbar und können den Zusatz von Tensiden erforderlich machen.

Das zum Anfeuchten verwendete Wasser sollte pharmazeutische Qualität haben. Es braucht nicht als solches zugesetzt zu werden, sondern kann als Träger für einen oder mehrere der Mischungsbestandteile dienen. Als Beispiel wurde schon die Wasserphase eines Latex des ionogenen Polymers genannt. Es kann gegebenenfalls auch die mitverwendete Säure oder Base oder den Wirkstoff selbst in gelöster oder suspendierter Form enthalten.

Die Umsetzung wird vorteilhaft bei Raumtemperatur durchgeführt. Geeignet ist der Temperaturbereich von 10 bis 30°C. Niedrigere Temperaturen sind nur angemessen, wenn es die Temperaturempfindlichkeit des Wirkstoffes erfordert. Höhere Temperaturen bis zu 60°C sind jedoch meist ohne Nachteil anwendbar. Sie können zum Beginn der Trocknung während der Umsetzung führen. Daher lassen sich bei dem Verfahren der Erfindung die Stufen der Umsetzung und der Trocknung meistens nicht deutlich voneinander unterscheiden. Allerdings sollte ein ausreichender Feuchtigkeitsgehalt solange aufrechterhalten werden, bis der erforderliche Umsetzungsgrad erreicht ist. Die Umsetzungsdauer kann zwischen wenigen Minuten und 24 Stunden liegen, vorzugsweise zwischen 10 und 20 Std. Zur Trocknung können erhöhte Temperaturen, beispielsweise bis zu 80°C angewendet werden. Die Trocknung kann durch Zufuhr von Warmluft, Absaugen der Dämpfe, Anlegen von Vakuum und Umwälzen des pulverförmigen Gemisches in einer Trocknungstrommel oder mittels einer geeigneten Rührvorrichtung beschleunigt werden. Sie kann in vielen Fällen bei einer Restfeuchte von z.B. 5 Gew.-% abgebrochen werden. Die Trocknungszeit liegt im allgemeinen zwischen 10 min und 48 Stunden. Das Trockengut fällt je nach der Art des verwendeten Trockners als Pulver, als Granulat oder in großen, unregelmäßig geformten Aggregaten an und kann gegebenenfalls mit einer Mühle auf die gewünschte Größe zerkleinert werden.

## BEISPIELE

### Beispiel 1

Geschmacksisolierung einer bitter schmeckenden Arzneistoffbase

In einer Salbenschale werden 20.0 g Atenolol mit 38.5 g eines sprühgetrockneten Emulsionspolymerisats aus 50 Gew.-% Methacrylsäure- und 50 Gew.-% Methylmethacrylat-Einheiten (Handelsprodukt EUDRAGIT L 100, Röhm GmbH) gemischt. Anschließend werden 25 g Wasser eingearbeitet. Es entsteht eine körnige Masse. Die so hergestellte Masse wird 24 Stunden bei 60°C im Trockenschrank getrocknet. Nach dem Trocknen wird der Wirkstoff-Polymer-Komplex auf eine Korngröße < 0.1 mm vermahlen.

Das so hergestellte Pulver schmeckt noch leicht bitter (Bitterwert nach DAB 9 zw. 1000 und 2000), und kann direkt zu neutral schmeckendem Sirup oder Tabletten weiterverarbeitet werden.

### Beispiel 2

Geschmacksisolierung einer bitter schmeckenden Arzneistoffbase

In einer Salbenschale werden 20.0 g Trimethoprim mit 35.3 g des im Beispiel 1 verwendeten Polymerisats gemischt. Anschließend werden 19 g Wasser gleichmäßig eingearbeitet. Es entsteht eine krümelige Masse. Die so hergestellte Masse wird 24 Stunden bei 60° C im Trockenschrank getrocknet. Nach dem Trocknen wird der Wirkstoff-Polymer-Komplex auf eine Korngröße < 0.1 mm vermahlen. Das so hergestellte Pulver schmeckt noch leicht bitter (Bitterwert nach DAB 9 < 1000), und kann direkt zu neutral schmeckendem Sirup oder Tabletten weiterverarbeitet werden.

### Beispiel 3

Geschmacksisolierung eines bitter schmeckenden Arzneistoffsalzes

In einer Salbenschale werden 15.0 g Metoclopramid-HCl mit 21.7 g des in Beispiel 1 verwendeten Polymerisats gemischt. Anschließend werden 25 g Wasser eingearbeitet. Es entsteht eine breiige Masse. Zu dieser Mischung gibt man unter Rühren eine Lösung von 3.1 g Natriumcarbonat in 15.0 g Wasser. Während der Reaktion entweicht $CO_2$. Die so hergestellte Masse wird 24 Stunden bei 60°C im Trockenschrank getrocknet. Nach dem Trocknen wird der Wirkstoff-Polymer-Komplex auf eine Korngröße < 0.1 mm vermahlen.

Das so hergestellte Pulver schmeckt noch leicht bitter (Bitterwert nach DAB 9 zw. 2000 und 5000), und kann direkt zu neutral schmeckendem Sirup oder Tabletten weiterverarbeitet werden.

### Beispiel 4

Geschmacksisolierung eines bitter schmeckenden Arzneistoffsalzes

In einer Salbenschale werden 20.0 g Pentoxyverincitrat mit 19.4 g des in Beispiel 1 verwendeten Polymerisats und 2.8 g Natriumcarbonat gemischt. Anschließend werden 30 g Wasser eingearbeitet. Es entsteht eine breiige Masse. Während der Reaktion entweicht $CO_2$. Die so hergestellte Masse wird 24 Stunden bei 60°C im Trockenschrank getrocknet. Nach dem Trocknen wird der Wirkstoff-Polymer-Komplex auf eine Korngröße < 0.1 mm vermahlen.

Das so hergestellte Pulver schmeckt leicht sauer, jedoch nicht bitter (Bitterwert nach DAB 9 < 1000), und kann direkt zu neutral schmeckendem Sirup oder Tabletten weiterverarbeitet werden.

### Beispiel 5

Geschmacksisolierung eines bitter schmeckenden Arzneistoffsalzes

In einer Salbenschale werden 20.0 g Propranolol-HCl mit 34.6 g des in Beispiel 1 verwendeten Polymerisats gemischt. Anschließend werden 30 g Wasser eingearbeitet. Es entsteht eine körnige Masse. Zu dieser Mischung gibt man unter Rühren eine Lösung von 5.0 g Natriumcarbonat in 15.0 g Wasser. Während der Reaktion entweicht $CO_2$. Die so hergestellte Masse wird 24 Stunden bei 60°C im Trockenschrank getrocknet. Nach dem Trocknen wird der Wirkstoff-Polymer-Komplex auf eine Korngröße < 0.1 mm vermahlen.

Das so hergestellte Pulver schmeckt neutral (Bitterwert nach DAB 9 < 1000), und kann direkt zu neutral schmeckendem Sirup oder Tabletten weiterverarbeitet werden.

Beispiel 6

Geschmacksisolierung eines bitter schmeckenden Arzneistoffsalzes

In einem Universalmischer Stephan UMC 12 werden 735.0 g Verapamil-HCl mit 765.0 g des in Beispiel 1 verwendeten Polymerisats gemischt. Anschließend werden 1125 g Wasser eingearbeitet. Es entsteht eine breiige Masse. Zu dieser Mischung gibt man unter Rühren eine Lösung von 95.3 g Natriumcarbonat in 400.0 g Wasser. Während der Reaktion entweicht $CO_2$. Die so hergestellte Masse wird 24 Stunden bei 60°C im Trokkenschrank getrocknet. Nach dem Trocknen wird der Wirkstoff-Polymer-Komplex auf eine Korngröße < 0.1 mm vermahlen. Das so hergestellte Pulver schmeckt neutral (97 % ige Bindung), und kann direkt zu neutral schmeckendem Sirup oder Tabletten weiterverarbeitet werden.

Beispiel 7

Geschmacksisolierung eines bitter schmeckenden Arzneistoffsalzes

In einem Universalmischer Stephan UMC 12 werden 735.0 g Verapamil-HCl mit 2550.0 g einer 30 %igen wäßrigen Dispersion eines Emulsionspolymerisats aus 50 Gew.-% Methacrylsäure- und 50 Gew.-% Ethylacrylat-Einheiten (Handelsprodukt EUDRAGIT L 30 D, Röhm GmbH) durchfeuchtet. Es entsteht eine breiige Masse. Zu dieser Mischung gibt man unter Rühren eine Lösung von 95.3 g Natriumcarbonat in 400.0 g Wasser. Während der Reaktion entweicht $CO_2$. Die so hergestellte Masse wird 48 Stunden bei 60°C im Trockenschrank getrocknet. Nach dem Trocknen wird der Wirkstoff-Polymer-Komplex auf eine Korngröße < 0.1 mm vermahlen.

Das so hergestellte Pulver schmeckt neutral (97 % ige Bindung), und kann direkt zu neutral schmeckendem Sirup oder Tabletten weiterverarbeitet werden.

Beispiel 8

Retardierung eines Arzneistoffsalzes

In einem Universalmischer Stephan UMC 12 werden 735.0 g Verapamil-HCl mit 765.0 g des in Beispiel 1 verwendeten Polymerisats gemischt. Anschließend werden 1125 g Wasser eingearbeitet. Es entsteht eine breiige Masse. Zu dieser Mischung gibt man unter Rühren eine Lösung von 95.3 g Natriumcarbonat in 400.0 g Wasser. Während der Reaktion entweicht $CO_2$. Die so hergestellte Masse wird 24 Stunden bei 60°C im Trokkenschrank getrocknet. Nach dem Trocknen wird der Wirkstoff-Polymer-Komplex mit einem Siebgranulator der Maschenweite 1.2 mm (Fa. Seidenader) zerkleinert und eine Teilchengrößenfraktion von 0.3 - 1.2 mm ausgesiebt.

Das so hergestellte Granulat zeigt eine verzögerte Wirkstoffabgabe (USP XXI, Paddle, 100 UpM, 37 C, 2 Stunden pH 1.0 anschließend pH 6.8) über 6 Stunden. Die Wirkstoffabgaben der einzelnen Kornfraktionen sind in Abb.2 angegeben.

Beispiel 9

Herstellung eines geschmacksneutralen Verapamil-Sirups

In einem Rührkessel werden 1279.0 g Saccharose mit 1139.9 g Wasser auf ca. 50 C erhitzt und bis zur vollständigen Lösung langsam gerührt. Nach dem Abkühlen auf ca. 20°C gibt man 38.0 g Verapamil-Salz (aus Beispiel 6) zu und homogenisiert ca. 10 Minuten. Zu der Suspension gibt man nun unter Rühren 27.3 g einer Mischung aus microkristalliner Cellulose und Na-Carboxymethylcellulose (Avicel RC 591$^{R}$) und 2.8 g Keltrol$^{R}$ F (Polysaccharid B 1459) und homogenisiert ca. 15 Minuten.

Der so hergestellte Sirup ist eine homogene, weiße Flüssigkeit mit süßem Geschmack. Er hat eine Viskosität von ca. 2000 mPa·s und eine Dichte von ca. 1.25 g/ml. Der Wirkstoffgehalt beträgt 8 mg Verapamil (berechnet als Base)/ml. Die Wirkstofffreisetzung im Test nach USP XXI (Paddle, 100 UPM, 37°C, pH 1.0) beträgt ca. 80% nach 60 Minuten.

Beispiel 10

Herstellung einer geschmacksneutralen Verapamil-Tablette

In einem Doppelkonusmischer werden 323.5 g Verapamil-Polymer-Komplex (aus Beispiel 6) mit 150.0 g microkristalliner Cellulose (Handelsprodukt Avicel pH 102) und 25.0 g Natriumcarboxymethylstärke (Handelsprodukt Explotab$^R$) 20 Minuten gemischt. Anschließend gibt man 1.5 g Magnesiumstearat zu und mischt weitere 10 Minuten. Diese Masse wird bei einer Preßkraft von 20 kN zu Tabletten mit einem Durchmesser von 8 mm, einem Wölbungsradius von 16 mm und einem Sollgewicht von 280 mg (= 80 mg Verapamilbase) verpreßt. Die Zerfallszeit nach DAB 9 liegt unter 20 Minuten.

Beispiel 11

Geschmacksisolierung eines bitter schmeckenden Arzneistoffsalzes

In einer Salbenschale werden 20.0 g Pentoxyverincitrat mit 14.5 g eines pulverförmigen Mischpolymerisats aus 67 Gew.-% Methacrylsäure- und 33 Gew.-% Methylmethacrylat-Einheiten (Handelsprodukt EUDISPERT hv, Röhm GmbH) gemischt. Anschließend werden 20 g Wasser eingearbeitet. Es entsteht eine körnige Masse. Zu dieser Mischung gibt man unter Rühren eine Lösung von 2.8 g Natriumcarbonat in 15.0 g Wasser. Während der Reaktion entweicht $CO_2$. Die so hergestellte Masse wird 24 Stunden bei 60°C im Trockenschrank getrocknet. Nach dem Trocknen wird der Wirkstoff-Polymer-Komplex vermahlen (Korngröße < 0.1 mm).

Das so hergestellte Pulver schmeckt leicht sauer, jedoch nicht bitter (Bitterwert nach DAB 9 < 1000), und kann direkt zu geschmacksneutralem Sirup oder Tabletten weiterverarbeitet werden.

Beispiel 12

Geschmacksisolierung eines bitter schmeckenden Arzneistoffsalzes

In einer Salbenschale werden 20.0 g Pentoxyverincitrat mit 32.7 g eines sprühgetrockneten Emulsionspolymerisats aus 70 Gew.-% Methylmethacrylat- und 30 Gew.-% Methacrylsäure-Einheiten (Handelsprodukt EUDRAGIT S 100) gemischt. Anschließend werden 30 g Wasser eingearbeitet. Es entsteht eine körnige Masse. Zu dieser Mischung gibt man unter Rühren eine Lösung von 2.8 g Natriumcarbonat in 15.0 g Wasser. Während der Reaktion entweicht $CO_2$. Die so hergestellte Masse wird 24 Stunden bei 60°C im Trockenschrank getrocknet. Nach dem Trocknen wird der Wirkstoff-Polymer-Komplex vermahlen (Korngröße < 0.1 mm). Das so hergestellte Pulver schmeckt leicht sauer, jedoch nicht bitter (Bitterwert nach DAB 9 < 1000), und kann direkt zu geschmacksneutralem Sirup oder Tabletten weiterverarbeitet werden.

Beispiel 13

Geschmacksisolierung eines bitter schmeckenden Arzneistoffsalzes

In einer Salbenschale werden 20.0 g Pentoxyverincitrat mit 19.4 g des in Beispiel 1 verwendeten Polymerisats gemischt. Anschließend werden 25 g Wasser eingearbeitet. Es entsteht eine breiige Masse. Zu dieser Mischung gibt man unter Rühren eine Lösung von 4.4 g Natriumhydrogencarbonat in 15.0 g Wasser. Während der Reaktion entweicht $CO_2$. Die so hergestellte Masse wird 24 Stunden bei 60°C im Trockenschrank getrocknet. Nach dem Trocknen wird der Wirkstoff-Polymer-Komplex vermahlen (Korngröße < 0.1 mm). Das so hergestellte Pulver schmeckt leicht sauer, jedoch nicht bitter (Bitterwert nach DAB 9 < 1000), und kann direkt zu geschmacksneutralem Sirup oder Tabletten weiterverarbeitet werden.

Beispiel 14

Geschmacksisolierung eines bitter schmeckenden Arzneistoffsalzes

In einer Salbenschale werden 20.0 g Propranolol-HCl mit 34.6 g eines sprühgetrockneten Emulsionspolymerisats aus 50 Gew.-% Ethylacrylat- und 50 Gew.-% Methacrylsäure-Einheiten (Handelsprodukt EUDRAGIT L 100-55, Röhm GmbH) gemischt. Anschließend werden 30 g Wasser eingearbeitet. Es entsteht eine körnige Masse. Zu dieser Mischung gibt man unter Rühren eine Lösung von 5.0 g Natriumcarbonat in 15.0 g Wasser. Während der Reaktion entweicht $CO_2$. Die so hergestellte Masse wird 24 Stunden bei 60°C im Trockenschrank getrocknet. Nach dem Trocknen wird der Wirkstoff-Polymer-Komplex auf eine Korngröße < 0.1 mm vermahlen. Das so hergestellte Pulver schmeckt neutral (Bitterwert nach DAB 9 < 1000), und kann direkt zu neutral schmeckendem Sirup oder Tabletten weiterverarbeitet werden.

Beispiel 15

Geschmacksisolierung eines bitter schmeckenden Arzneistoffsalzes

In einer Salbenschale werden 20.0 g Propranolol-HCl mit 17.3 g schwach vernetzter Polyacrylsäure mit einem MG von etwa 3 Millionen (Handelsprodukt CARBOPOL$^R$ 934 P) gemischt. Anschließend werden 20 g Wasser eingearbeitet. Es entsteht eine körnige Masse. Zu dieser Mischung gibt man unter Rühren eine Lösung von 5.0 g Natriumcarbonat in 15.0 g Wasser. Während der Reaktion entweicht $CO_2$. Die so hergestellte Masse wird 24 Stunden bei 60°C im Trockenschrank getrocknet. Nach dem Trocknen wird der Wirkstoff-Polymer-Komplex auf eine Korngröße < 0.1 mm vermahlen. Das so hergestellte Pulver schmeckt neutral (Bitterwert nach DAB 9 < 1000), und kann direkt zu neutral schmeckendem Sirup oder Tabletten weiterverarbeitet werden.

Beispiel 16

Geschmacksisolierung eines bitter schmeckenden Arzneistoffsalzes

In einer Salbenschale werden 15.0 g Metoclopramid-HCl mit 30.0 g des in Beispiel 1 verwendeten Polymerisats gemischt. Anschließend werden 25 g Wasser eingearbeitet. Es entsteht eine körnige Masse. Zu dieser Mischung gibt man unter Rühren eine Lösung von 3.1 g Natriumcarbonat in 15.0 g Wasser. Während der Reaktion entweicht $CO_2$. Die so hergestellte Masse wird 24 Stunden bei 60°C im Trockenschrank getrocknet. Nach dem Trocknen wird der Wirkstoff-Polymer-Komplex auf ein Korngröße < 0.1 mm vermahlen. Das so hergestellte Pulver schmeckt neutral (Bitterwert nach DAB 9 < 1000), und kann direkt zu neutral schmeckendem Sirup oder Tabletten weiterverarbeitet werden.

## Patentansprüche

1. Verfahren zur Herstellung eines komplexierten Arzneimittels aus einem ionogenen Wirkstoff durch Umsetzung des Wirkstoffes mit einem komplementär ionogenen partikelförmigen Polymeren,
dadurch gekennzeichnet, daß man den ionogenen Wirkstoff und das Komplementär ionogene partikelförmige Polymere mit einer lediglich zum Aufeuchten ausreichenden Wassermenge zu einem pulverförmigen bis breiartigen Gemisch anteigt und das so erhaltene Gemisch Hocknet.

2. Verfahren nach Anspruch 1, dadurch gekennzeichnet, daß der Wirkstoff in Form eines Salzes mit einem organischen oder anorganischen Gegenion eingesetzt wird und daß der Mischung zusätzlich eine niedermolekulare Säure oder Base zur Neutralisation des Gegenions des Wirkstoffes zugesetzt wird.

3. Verfahren nach Anspruch 1 oder 2, dadurch gekennzeichnet, daß als Wirkstoff eine basische organische Stickstoffverbindung oder deren Salz mit einer niedermolekularen Säure eingesetzt wird.

4. Verfahren nach einem oder mehreren der Ansprüche 1 bis 3, dadurch gekennzeichnet, daß das Polymer in Form einer wäßrigen Dispersion eingesetzt wird und der Wassergehalt der Dispersion der zum Anfeuchten des Gemisches erforderlichen Wassermenge entspricht.

5. Verfahren nach einem oder mehreren der Ansprüche 1 bis 4, dadurch gekennzeichnet, daß als Polymer ein Mischpolymerisat aus 10 bis 90 Gew.-% einer äthylenisch ungesättigten, radikalisch polymerisierbaren Carbonsäure mit wenigstens einem nichtionogenen, wasserunlöslichen äthylenisch ungesättigten, radikalisch polymerisierbaren Comonomer verwendet wird.

6. Verfahren nach einem oder mehreren der Ansprüche 1 bis 5, dadurch gekennzeichnet, daß als partikelförmiges Polymer ein sprühgetrocknetes Emulsionspolymerisat verwendet wird.

7. Verfahren nach einem oder mehreren der Ansprüche 1 bis 5, dadurch gekennzeichnet, daß als partikelförmiges Polymer ein Perlpolymerisat verwendet wird.

8. Verfahren nach einem oder mehreren der Ansprüche 1 bis 7, dadurch gekennzeichnet, daß der ionogene Wirkstoff eine Säure und das partikelförmige Polymere als komplementär ionogene Gruppen seitenständige Aminogruppen enthält.

## Claims

1. A method for preparing a complexed pharmaceutical composition from an ionogenic active substance by reacting the active substance with a complementary ionogenic particle-like polymer, characterised in that the ionogenic active substance and the complementary ionogenic particle-like polymer are mixed to form a paste with an amount of water which simply suffices for wetting, forming a powder-like to paste-like mixture and the mixture thus obtained is dried.

2. A method according to claim 1, characterised in that the active substance is used in the form of a salt with an organic or inorganic counterion and that a low-molecular acid or base is also added to the mixture in order to neutralise the counterion of the active substance.

3. A method according to claim 1 or 2, characterised in that a basic organic nitrogen compound or a salt thereof is used with a low-molecular acid as the active substance.

4. A method according to one or more of claims 1 to 3, characterised in that the polymer is used in the form of an aqueous dispersion and the water content of the dispersion corresponds to the amount of water required for wetting the mixture.

5. A method according to one or more of claims 1 to 4, characterised in that a copolymer of 10 to 90 wt.% of an ethylene-unsaturated, radically polymerisable carboxylic acid is used with at least one non-ionogenic, water-insoluble ethylene-unsaturated, radically polymerisable comonomer as the polymer.

6. A method according to one or more of claims 1 to 5, characterised in that a spray-dried emulsion polymer is used as the particle-like polymer.

7. A method according to one or more of claims 1 to 5, characterised in that a bead polymer is used as the particle-like polymer.

8. A method according to one or more of claims 1 to 7, characterised in that the ionogenic active substance contains an acid and the particle-like polymer contains side amino groups as complementary ionogenic groups.

## Revendications

1. Procédé de préparation d'un médicament complexé à partir d'une substance active ionogène, par réaction de la substance actlve avec un polymère ionogène complémentaire en particules, caractérisé en ce qu'on transforme la substance actlve ionogène et le polymère ionogène complémentaire en particules, en utilisant la quantité d'eau strictement nécessaire pour l'humectation, en un mélange pulvérulent à pâteux et on sèche le mélange ainsi obtenu.

2. Procédé selon la revendication 1, caractérisé en ce que la substance active est mise en oeuvre sous la forme d'un sel avec un ion opposé organique ou inorganique, et en ce qu'il est ajouté en plus au mélange un acide ou une base de faible poids moléculaire pour la neutralisation de l'ion opposé de la substance active.

3. Procédé selon la revendication 1 ou 2, caractérisé en ce qu'on met en oeuvre, comme substance active, un composé organique azoté ou un sel de celui-ci avec un acide de faible poids moléculaire.

4. Procédé selon l'une quelconque des revendications 1 à 3, caractérisé en ce que le polymère est mis en oeuvre sous forme d'une dispersion aqueuse et en ce que la teneur en eau de la dispersion correspond à la quantité d'eau nécessaire pour l'humectation du mélange.

5. Procédé selon l'une quelconque des revendications 1 à 4, caractérisé en ce qu'on utilise, comme polymère, un copolymère de 10 à 90% en poids d'un acide carboxylique à insaturation éthylénique, susceptible de polymérisation radicalaire, et d'au moins un comonomère à insaturation éthylénique, susceptible de polymérisation radicalaire, insoluble dans l'eau et non ionogène.

6. Procédé selon l'une quelconque des revendications 1 à 5, caractérisé en ce qu'on utilise, comme polymère en particules, un produit de polymérisation en émulsion séché par pulvérisation.

7. Procédé selon l'une quelconque des revendications 1 à 5, caractérisé en ce qu'on utilise, comme polymère en particules, un produit de polymérisation en perles.

8. Procédé selon l'une quelconque des revendications 1 à 7, caractérisé en ce que la substance active ionogène contient un acide et le polymère en particules contient, en tant que groupements ionogènes complémentaires, des groupements amino latéraux.

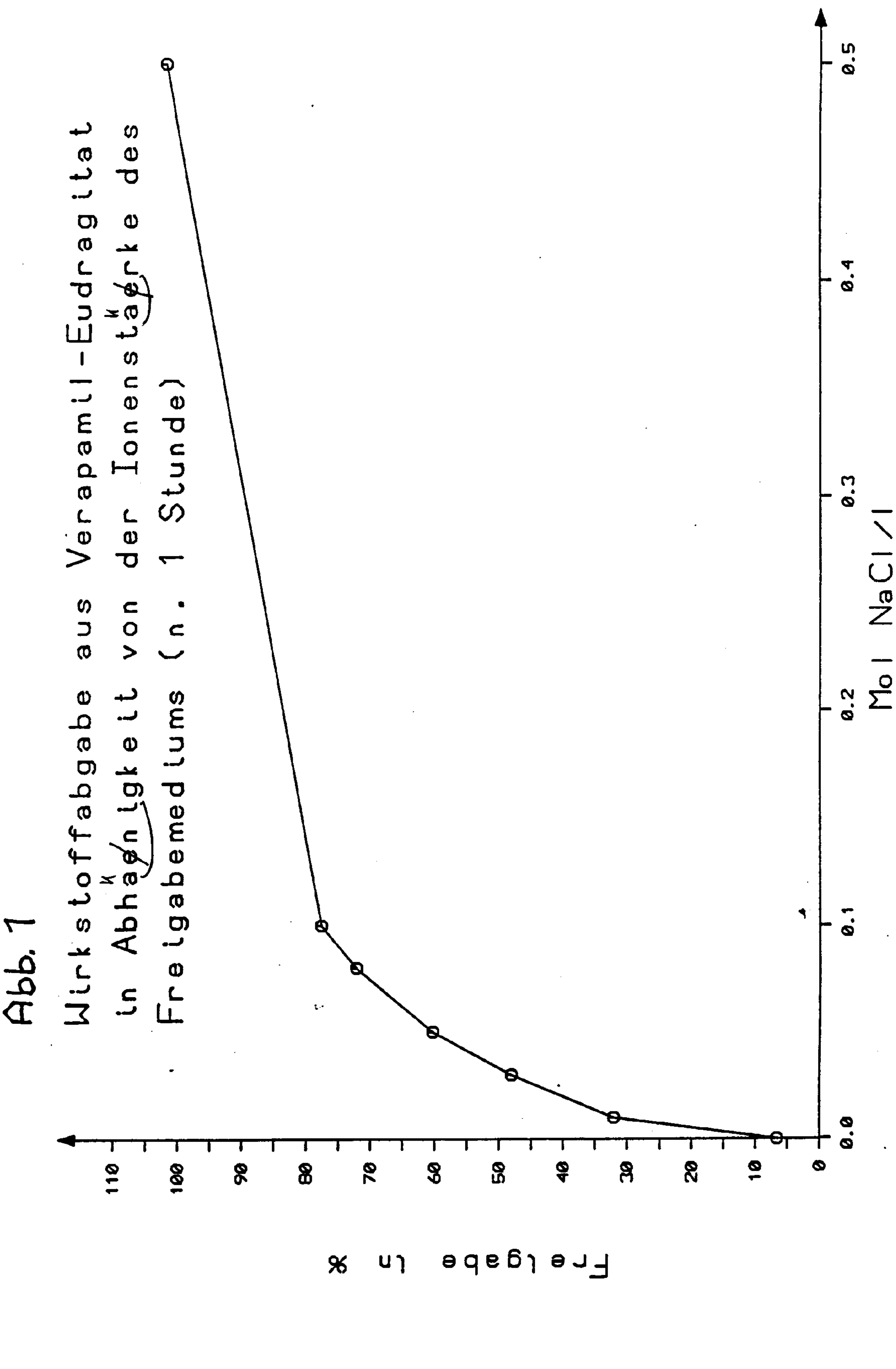
Abb. 1
Wirkstoffabgabe aus Verapamil-Eudragitat in Abhaenigkeit von der Ionenstaerke des Freigabemediums (n. 1 Stunde)
Freigabe in %
0
10
20
30
40
50
60
70
80
90
100
110
0.0
0.1
0.2
0.3
0.4
0.5
Mol NaCl / l

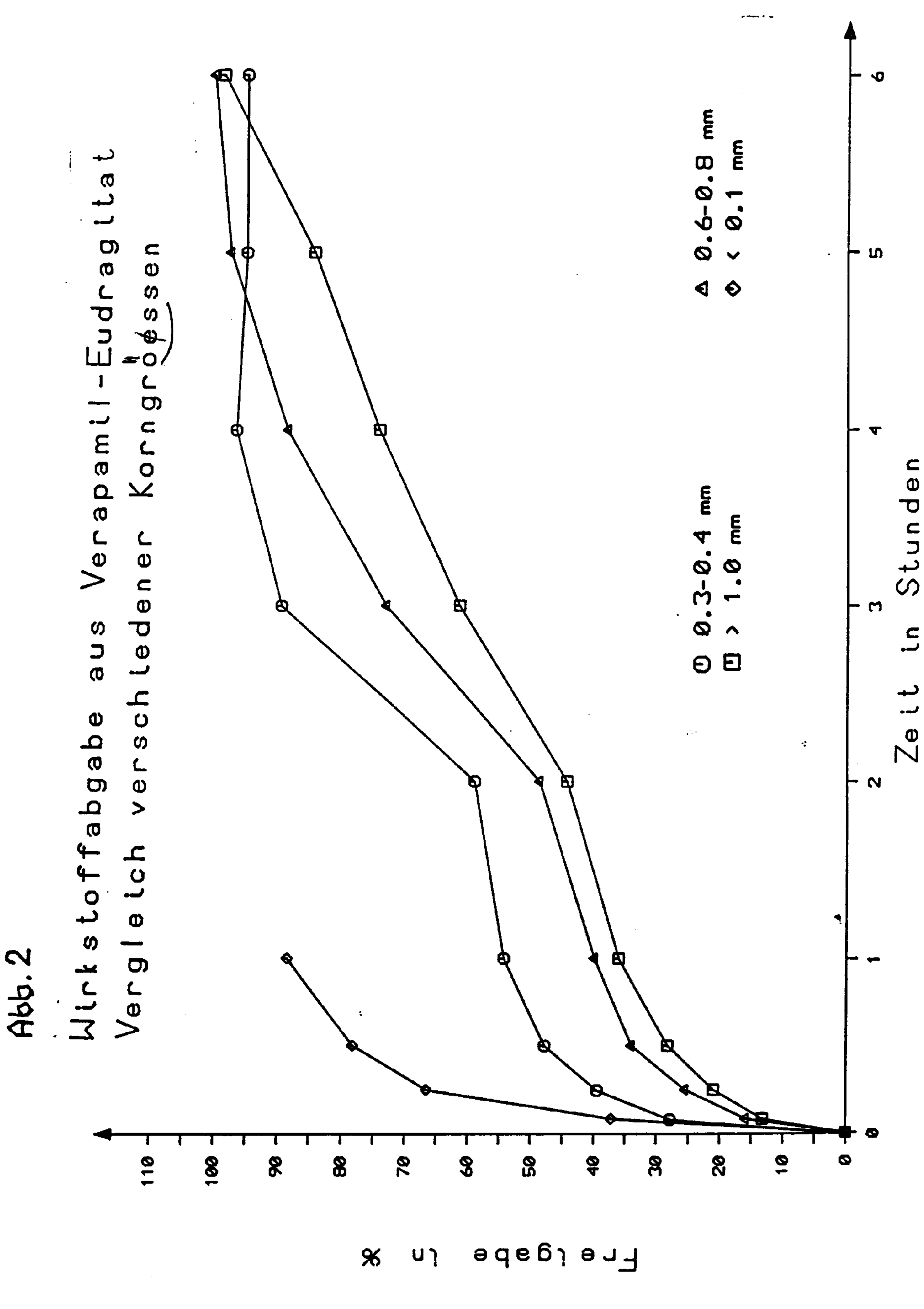

Abb.2
Wirkstoffabgabe aus Verapamil-Eudragitat
Vergleich verschiedener Korngrössen
0.3-0.4 mm
> 1.0 mm
0.6-0.8 mm
< 0.1 mm
Freigabe in %
0
10
20
30
40
50
60
70
80
90
100
110
Zeit in Stunden
0
1
2
3
4
5
6